# EUROPEAN PATENT APPLICATION

(11) **EP 4 497 845 A1**
(43) Date of publication of application: **29.01.2025**
(21) Application number: 23775267.0
(22) Date of filing: 21.03.2023
(51) Int. Cl.: C25B 3/05, C25B 3/23, C07D 317/36, C07D 317/38, C25B 3/27

(54) **METHOD FOR ELECTROCHEMICALLY SYNTHESIZING ALKYLENE CARBONATE**

(30) Priority: 22.03.2022 KR 20220035181
(71) Applicant: Seoul National University R&DB Foundation, Seoul 08826 (KR)
(72) Inventor: NAM, Ki-Tae, Seoul 06102 (KR); JANG, Jun-Ho, Seoul 08814 (KR)
(74) Representative: Maiwald GmbH
(86) International application number: PCT/KR2023/003719
(87) International publication number: WO 2023/182771

(57) **Abstract**

The present invention relates to a method for synthesizing alkylene carbonate, that is, alkylene carbonate including ethylene carbonate and propylene carbonate, and, more specifically, to a method for electrochemically synthesizing alkylene carbonate, the method comprising: a first step of obtaining a mixture of a halogen-substituted nitrogen compound (XY), a nitrogen compound (Y), and hydroxide ions (OH⁻) by electrochemically reacting halogen and a nitrogen compound in a reactor provided with a negative electrode and a positive electrode and including a solvent; and a second step of obtaining alkylene carbonate by mixing the mixture with alkylene and carbon dioxide.

## Description

### Technical Field

The present disclosure relates to a method for synthesizing alkylene carbonate, and more specifically, to a method for synthesizing alkylene carbonate, such as ethylene carbonate and propylene carbonate using an electrochemical method.

### Background Art

Alkylene carbonate is not only used as a raw material for polycarbonate, a pharmaceutical intermediate, an oxyalkylation agent in a dye synthesis process, a process equipment protector, a solvent in a process for producing textiles, and the like, but the scope of use thereof has been recently expanded, including as a solvent for a polymer electrolyte in a secondary battery.

The conventionally known synthesis of ethylene carbonate and propylene carbonate was undertaken by reacting carbon dioxide with ethylene oxide or propylene oxide in the presence of a catalyst, but in this case, in order for the accompanying reaction to achieve an industrially useful reaction rate, high temperature and high pressure carbon dioxide gas or liquid was required. In addition, in the case of ethylene oxide, there was a problem in that a large amount of carbon dioxide is released during the process of synthesizing ethylene oxide from ethylene. Since ethylene oxide or propylene oxide tends to decompose or polymerize under high temperature and pressure conditions, various catalysts have been developed to alleviate reaction conditions.

For example, Japanese Patent Laid-Open No. 9-67365 discloses a method of using Kl (potassium iodide) as a catalyst, and Japanese Patent Publication No. 59-13776 discloses a method of using a tetraalkyl phosphnium halide such as tributylmethyl phosphonium iodide as a catalyst, respectively. These patents state that a yield of 50 to 95% is obtained when reacted at a temperature of 100 to 170°C for 1 to 5 hours, but in order to obtain a high yield, there is a problem in that the reaction should be performed at a high temperature for a long period of time, and a content of moisture of carbon dioxide and alkylene oxide, which are raw materials, should be controlled to be below several hundred ppm. Accordingly, a method using an ion exchange resin as a catalyst has been proposed, but this method has the problem of a low yield at a temperature of 80 to 100°C.

In summary, the conventional technology for commercially producing ethylene carbonate and propylene carbonate has difficult reaction conditions, including that high-pressure gas or liquid carbon dioxide should be reacted at a high temperature and, the content of moisture of the raw material should be very low, and the like. In addition, despite the development of various catalysts, the generation of carbon dioxide in the process of producing ethylene oxide cannot be avoided.

Accordingly, under more alleviated conditions, when a technology that does not require the use of high-pressure carbon oxide, ethylene oxide, propylene oxide, and the like, and can obtain carbonate in a high concentration, so the technology has a simple subsequent purification process, is developed, it is expected that the technology will be widely applied in the related fields.

### Summary of Invention

### Technical Problem

An aspect of the present disclosure is to provide a method for synthesizing alkylene carbonate using an electrochemical process.

### Solution to Problem

According to an aspect of the present disclosure, a method for electrochemically synthesizing alkylene carbonate is provided, the method including: a first operation of obtaining a mixture of a halogen-substituted nitrogen compound (XY), a nitrogen compound (Y), and hydroxide ions (OH-), by electrochemically reacting halogen and a nitrogen compound, in a reactor having cathode and anode electrodes, and including a solvent; and a second operation of obtaining alkylene carbonate by mixing the mixture with alkylene (CₓH₂ₓ, where x is an integer of 2 to 10) and carbon dioxide.

### Advantageous Effects of Invention

According to the present disclosure, alkylene carbonate such as ethylene carbonate, propylene carbonate, and the like, may be provided in a high yield through an electrochemical process, the additional use of ingredients such as alkylene oxide is not required through an electrochemical process of alkylene. In addition, according to the present disclosure, a carbon dioxide capturing process in which a nitrogen compound and a hydroxide salt react with carbon dioxide, is included, making it possible to capture and additionally use carbon dioxide. In addition, as compared to the existing thermochemical process, the electrochemical-based carbonate synthesis of the present disclosure may be used in conjunction with renewable electric energy and may be presented as a more environmentally friendly synthesis process.

### Brief description of drawings

FIG. 1 is a graph illustrating a change in products over time when synthesizing ethylene carbonate according to the present disclosure.
FIG. 2 is a graph illustrating a change in products depending on current when synthesizing ethylene carbonate according to the present disclosure.
FIG. 3 is a graph illustrating a change in products depending on temperature when synthesizing ethylene carbonate according to the present disclosure.
FIG. 4 is a graph illustrating a change in hydrogen conversion efficiency at an anode(+) depending on current when synthesizing ethylene carbonate according to the present disclosure.
FIG. 5 graphically illustrates a yield of ethylene carbonate according to the type of nitrogen compound.
FIG. 6 illustrates the results of testing an amount of carbon dioxide that can be captured in a process for synthesizing ethylene carbonate according to the present disclosure.

### Best Mode for Invention

Hereinafter, preferred embodiments of the present disclosure will be described with reference to the attached drawings. However, the embodiments of the present disclosure may be modified to have various other forms, and the scope of the present disclosure is not limited to the embodiments described below.

According to the present disclosure, a method for synthesizing alkylene (alkene) carbonate by an electrochemical method is provided, and in more detail, the method for electrochemically synthesizing alkylene carbonate of the present disclosure includes: a first operation of obtaining a mixture of a halogen-substituted nitrogen compound (XY), a nitrogen compound (Y), and hydroxide ions (OH⁻), by electrochemically reacting halogen and a nitrogen compound, in a reactor having cathode and anode electrodes, and including a solvent; and a second operation of obtaining alkylene carbonate by mixing the mixture with alkylene (CₓH₂ₓ, where x is an integer of 2 to 10) and carbon dioxide.

As an electrolyte used in the electrochemical reaction of the present disclosure, various liquid electrolytes, such as mixed salts or ionic salts, may be used, and the mixed salts include halogen anions such as F⁻, Cl-, Br-, I-, and the like. Specifically, NaCl, NaBr, NaI, and the like may be used in combination with water-soluble salt and ionic liquid, but the present disclosure is not limited thereto.

In this case, metal or a metal composite used as the cathode electrode should not react with the liquid electrolyte such as the mixed salt, an ionic salt, or the like, and should not have any effect on corrosion or impurities.

The anode and cathode electrodes are not particularly limited, but include, for example, Fe, Al, Ni, stainless steel 304 (SUS 304), a Dimensionally Stabilized Anode (DSA) electrode, a Ru oxide-based composition electrode, an Ir oxide-based composition electrode, and the like can be used, and in more detail, the anode(+) electrode is a metal oxide or an oxide alloy, preferably a nickel-based electrode such as an oxide of Ni-Fe, Ni-Co, or Ni-Mo alloy, and the cathode (-) electrode is preferably a DSA electrode. A DSA electrode has the advantage of having excellent characteristics as an electrode, such as having a relatively low overvoltage against oxygen generation and being able to maintain soundness for a very long time as compared to metal electrodes. For example, a DSA electrode may be a platinum-coated electrode commonly used in the plating industry.

A size of a reactor is not particularly limited, and the narrower the gap between the anode and cathodes, the lower an amount of operating resistance, which may increase energy efficiency, but the gap therebetween is not limited. In addition, a power supply device that can be used in the present disclosure is not particularly limited.

The electrochemical reaction in the first operation may be performed under conditions of 1 mA/cm² to 100 A/cm², and may be performed under conditions of, for example, 10 mA/cm² to 1 A/cm². When the current density is less than 1 mA/cm², there is a problem of slow reaction speed, and when the current density exceeds 100 A/cm², there is a problem of reduced efficiency due to an oxygen generation reaction at the cathode and instability of the cathode electrode.

In the first operation, a mixture of a halogen-substituted nitrogen compound (XY) and a nitrogen compound (Y) or a mixture of a halogen-substituted nitrogen compound (XY), a nitrogen compound (Y), and hydroxyl ions (OH⁻) are obtained according to Scheme 1-1, or Scheme 1-1 and Scheme 1-2. Regarding Scheme 1-2, when the solvent includes water, a nitrogen compound (Y⁻) reacts with water and hydroxide ions (OH⁻) may exist together, according to Scheme 1-2.

[Scheme 1-1] 4X- + 4HY = 2XY + 2H₂ + 2X- + 2Y-

[Scheme 1-2] 2Y- + 2H₂O = 2OH⁻ + 2HY

In Scheme 1-1, X is halogen selected from the group consisting of fluorine, chlorine, bromine and iodine, and in Schemes 1-1 and 1-2, Y is a nitrogen compound, and the nitrogen compound is a neutral nitrogen compound or an anionic nitrogen compound. Meanwhile, when water is present in the solvent, water may participate in the reaction and generate hydroxyl ions as shown in Scheme 1-2. In this case, an amount of hydroxide ions may be determined by an amount of conjugate acid (HY) of the nitrogen compound and pKa.

In the second operation, by mixing a mixture of a halogen-substituted nitrogen compound and a nitrogen compound or a mixture of a halogen-substituted nitrogen compound, a nitrogen compound, and hydrogen ions with alkylene (CₓH₂ₓ, where x is an integer of 2 to 10) and carbon dioxide, obtain halogenated alcohol and a hydrogen carbonateions (HCO₃⁻) from these by the following reaction of Schemes 2 and 3-1, respectively, and obtain alkylene carbonate from these by the following Scheme 4. Meanwhile, according to Scheme 1-2, when hydroxide ions are formed, the hydroxide ions can also be converted to hydrogen carbonate ions based on Scheme 3-2.

[Scheme 2] 2alkene + 2XY + 2H₂O = 2halogenated alcohol + 2HY

[Scheme 3-1] 2Y- + 2CO₂ + 2H₂O = 2HY + 2HCO₃⁻

[Scheme 3-2] 2OH- + 2CO₂ = 2HCO₃⁻

[Scheme 4] 2HCO₃⁻ + 2halogenated alcohol = 2alkylene carbonate + 2X- + 2H₂O

In Schemes 2 to 4, X and Y are as defined in Schemes 1-1 and 1-2, where alkylene may be selected from C₁-C₁₀ alkylene, is preferably selected from the group consisting of ethylene and propylene, where halogenated alcohol is selected from the group consisting of halogenated ethanol and halogenated propanol, and preferably, the alkylene carbonate is selected from the group consisting of ethylene carbonate and propylene carbonate.

The first operation and the second operation may be performed simultaneously or sequentially, and the first operation and the second operation may be performed in the same reactor or separately in several separate reactors, and the present disclosure is not limited thereto. When the first operation and the second operation are performed sequentially or in separate reactors, the reaction conditions of the first operation and the second operation can be set differently in more detail as needed, thereby improving the yield.

For example, in the second operation, ethylene and carbon dioxide may be mixed with the mixture of the halogen-substituted nitrogen compound, nitrogen compound, and hydroxyl ion in a separate operation or a separate reactor. When the mixing is performed in the separate reactor, for example, alkylene and carbon dioxide may be separately mixed in a plurality of reactors including the products obtained in the first operation, and the results obtained therefrom may be mixed again. In this case, Schemes 2, 3-1, and 3-2 below can occur in regions separated from each other.

[Scheme 2] 2alkene + 2XY + 2H₂O = 2halogenated alcohol + 2HY

[Scheme 3-1] 2Y- + 2CO₂ + 2H₂O = 2HY + 2HCO₃⁻

[Scheme 3-2] 2OH⁻ + 2CO₂ = 2HCO₃⁻

In this case, in Schemes 2, 3-1, and 3-2, X and Y are as defined in Schemes 1-1 and 1-2, where alkylene may be selected from C₂-C₁₀ alkylene, and is preferably selected from the group consisting of ethylene and propylene, where halogenated alcohol is selected from the group consisting of halogenated ethanol and halogenated propanol, and alkylene carbonate is selected from the group consisting of ethylene carbonate and propylene carbonate.

That is, in this case, halogenated alcohol, such as halogenated ethanol, halogenated propanol, and hydrogen carbonate ions (HCO₃⁻) may be obtained according to Schemes 2, 3-1, and 3-2, respectively, and ethylene carbonate, propylene carbonate, and the like can be obtained by using the halogenated ethanol, halogenated propanol, and hydrogen carbonate ions (HCO₃⁻) as reactants in the following Scheme 4 below.

[Scheme 4] 2HCO₃⁻ + 2halogenated alcohol = 2alkylene carbonate + 2X- + 2H₂O

In this case, in Scheme 4, X and Y are as defined in Schemes 1-1 and 1-2, and alkylene carbonate may be selected from the group consisting of ethylene carbonate and propylene carbonate.

Meanwhile, in the method for electrochemically synthesizing alkylene carbonate of the present disclosure, the first operation and/or the second operation may be performed at a temperature within a range of higher than 0°C and lower than or equal to 150°C, and may be performed, for example, at a temperature within a range of 1°C to 100°C, or 50°C to 100°C. When the temperature is below or equal to 0°C or above 150°C, there is a problem that the pressure should be increased because water, which is a solvent, freezes or changes phase into water vapor under atmospheric pressure.

The method for electrochemically synthesizing alkylene carbonate based on the present disclosure may be performed for 15 minutes to 24 hours, for example, 30 minutes to 6 hours, preferably 2 hours to 6 hours.

Meanwhile, when the second operation is performed separately, the operation may be performed at a temperature within a range of 40°C or higher and lower than 150°C, for example, at a temperature within a range of 45°C to 90°C, preferably at a temperature within a range of 60 to 90°C, and more preferably, may be performed at a temperature within a range of 65 to 85°C. When the temperature is below the above-described ranges, the reaction may be somewhat slow, and the temperature exceeds the range of the present disclosure, an amount of glycol generated increases due to an increase in side reactions as shown in the following Scheme 5, resulting in a problem of lowering the yield of carbonate.

HCO₃⁻ + halogenated alcohol = glycol + CO₂ + X- [Scheme 5]

In Scheme 5, X is as defined in Scheme 1-1. Halogenated alcohol may be selected, for example, from the group consisting of halogenated ethanol and halogenated propanol, and glycol may be selected from the group consisting of ethylene glycol and propylene glycol.

When the second operation is performed separately, the operation may be performed for 15 minutes to 24 hours, for example, for 30 minutes to 12 hours. When a reaction time is less than 15 minutes, a yield of a product may be insufficient, and when the reaction time exceeds 24 hours, the increase in the yield of the product due to the increase in reaction time is not significant, which is not preferable in terms of process economy.

The nitrogen compound (Y) of the present disclosure is preferably a nitrogen compound of which conjugate acid (HY) has a pKa of 7 to 14, preferably a pKa of 7 to 11, is preferably at least one selected from, for example, a compound having an imide functional group and an ammonium compound, and more specifically, as the compound having an imide function group, succinimide, hydantoin, cyanurate, and the like, may be used, and as the ammonium compound, ammonium, monoethanol ammonium, diethanol ammonium, and the like, may be used.

In the method for electrochemically synthesizing alkylene carbonate of the present disclosure, a solvent in the first step may be water, and the second operation may be performed in at least one solvent selected from the group consisting of polar organic solvents including water, alcohol, dimethylformamide, acetone, ether, and ester, or such solvent may be added during the second operation. For example, a solvent in the second operation may water, or an aqueous ethanol solution, etc.

Meanwhile, ethylene and carbon dioxide in the second operation may be mixed in a volume ratio or molar ratio of 1:0.5 to 2, and are preferably mixed in a ratio of 1:1.

Carbon dioxide and ethylene in the second operation may respectively be introduced to reach a saturated state in the solution, and the pressure can be increased to increase a saturation concentration. In addition, the introduction method is not limited, and may be introduced continuously, at regular intervals, or at required times, depending on the reaction.

When the ethylene and carbon dioxide are respectively introduced, these gases may be introduced by bubbling or by applying pressure using these gases. In this case, when pressure is applied in the second operation, the pressure range is from greater than 1 atm to 50 atm, for example, may be 2 to 4 atm, but is not limited thereto, and pressurization is not essential.

The method for electromechanically synthesizing alkylene carbonate of the present disclosure is performed not based on a gas phase reaction, but based on a gas-liquid reaction under a solvent, and alkylene carbonate may be synthesized using reactants that are difficult to convert to the gas phase.

Hereinafter, the present disclosure will be described in more detail through specific examples. The following examples are merely examples to aid understanding of the present disclosure, and the scope of the present disclosure is not limited thereto.

### Mode for Invention

### Example

### 1. Synthesis of ethylene carbonate

### Manufacturing Example 1

By using a porous nickel foam electrode as an anode (+), and using a DSA electrode as a cathode(-), two electrodes having a size of 0.5cm² were immersed in 10ml of electrolytic solution with 0.4M succinimide, and 1 M NaBr. A temperature of a vessel in which electrolysis was performed was set to be 90°C, and 4 atm of carbon dioxide and 2 atm of ethylene were applied. The two gases were introduced into a reaction vessel at 80 sccm and 40 sccm, respectively. Under the above conditions, a current of 400 mA/cm² was applied for a total charge of 192.97 C.

### 2. Confirmation of Product

### (1) Change in product over time

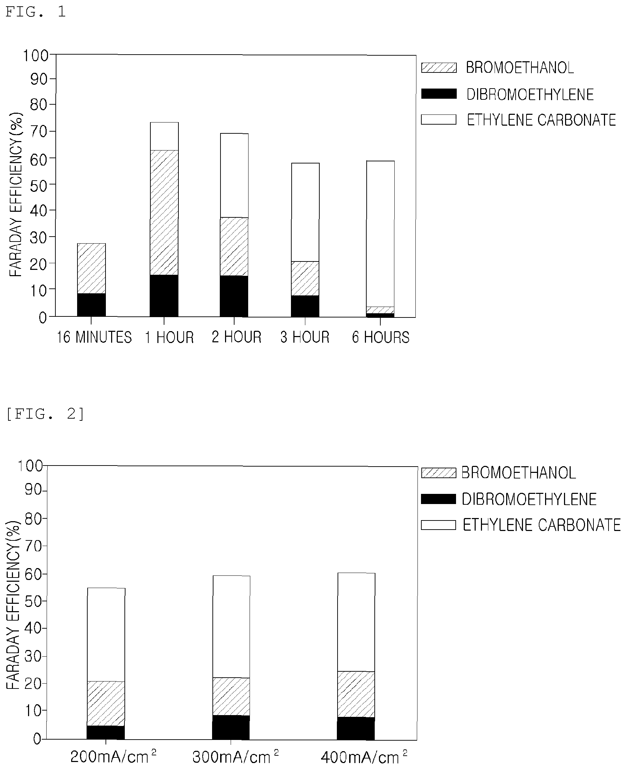

As a result of observing products in a solution over time when synthesizing ethylene carbonate by the same process as in Manufacturing Example 1, as can be seen in FIG. 1, it was confirmed that bromoethanol and dibromoethylene were initially produced, and then bromoethanol was converted to ethylene carbonate over time.

### (2) Change in product depending on current

When synthesizing ethylene carbonate using the same process as in Manufacturing Example 1, a total charge was fixed at 192.97 C, and the product in the solution was confirmed 3 hours after the start of the reaction at different current densities.

As a result thereof, as can be seen in FIG. 2, it was confirmed that ethylene carbonate can be formed in all current ranges of 100mA/cm² or more, which is industrially meaningful.

### (3) Change in product depending on temperature

When synthesizing ethylene carbonate using the same process as in Manufacturing Example 1, a product in the solution was confirmed 3 hours after the start of the reaction under different temperature conditions.

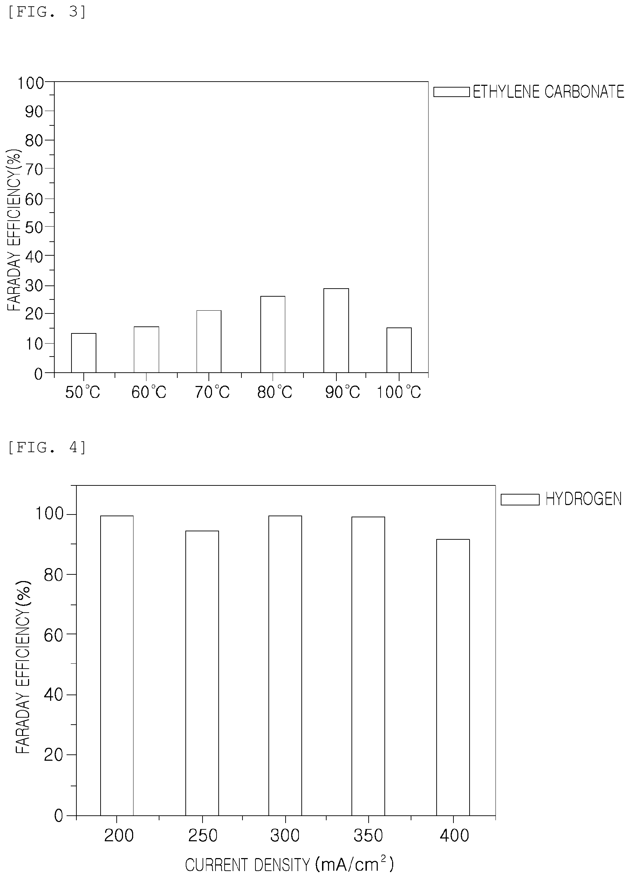

As a result thereof, as can be seen in FIG. 3, it was confirmed that excellent Faradaic efficiency was observed at a temperature within a range of 50 to 100 °C.

### 3. Confirmation of Reaction efficiency

### (1) Hydrogen conversion efficiency at an anode

The two electrodes in Manufacturing Example 1 were separated by an anion exchange membrane, and hydrogen production efficiency of the anode was measured under CO₂ bubbling conditions at room temperature and atmospheric pressure.

As a result thereof, as can be seen in FIG. 4, high hydrogen production efficiency was confirmed during the carbonate generation process.

### (2) Efficiency depending on type of nitrogen compound

When synthesizing ethylene carbonate using the same process as in Manufacturing Example 1, the production efficiency of ethylene carbonate in the solution was measured after 6 hours after the start of reaction according to the use of different nitrogen compounds. The pKa values of conjugate acid (HY) of the nitrogen compounds used in this experiment were shown in Table 1 below.

**[Table 1]**

| | Nitrogen compound | pKa |
|---|---|---|
| Example 1 | Succinimide | 9.5 |
| Example 2 | Hydantoin | 9.12 |
| Example 3 | Ethanolamine | 9.5 |
| Example 4 | Diethanolamine | 8.88 |
| Example 5 | Cyanurate | 11.4 |

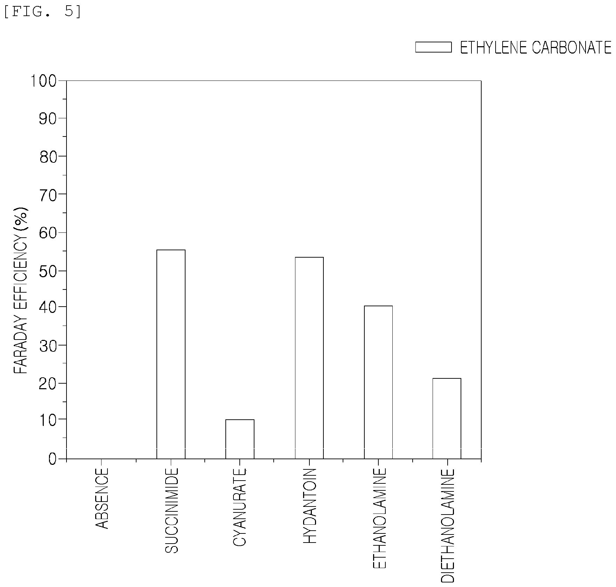

As a result thereof, as can be seen in FIG. 5, in the case of cyanurate with a relatively high pKa, it was confirmed that the production efficiency was somewhat reduced.

4. Experiment to confirm carbon dioxide capturing trend.

Each of 5ml of sodium hydroxide, sodium succinimide, and ethanolamine at a concentration of 0.5M were inserted in a vial with a capacity of 20ml, respectively, and carbon dioxide was supplied through bubbling. After carbon dioxide bubbling was performed until the pH of each solution no longer dropped, the amount of carbon dioxide captured in each solution was measured, and in this case, the amount of carbon dioxide generated after acid treatment of each solution was measured through a volume thereof.

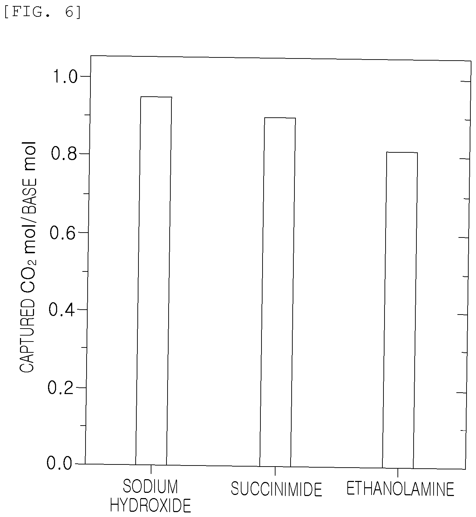

As a result thereof, as can be seen in FIG. 6, it was confirmed that about 0.9 molecules of carbon dioxide could be captured per succinimide anion molecule.

While example embodiments have been illustrated and described above, it will be apparent to those skilled in the art that modifications and variations could be made without departing from the scope of the present disclosure as defined by the appended claims.

## Claims

1. A method for electrochemically synthesizing alkylene carbonate, comprising:
a first operation of obtaining a mixture of a halogen-substituted nitrogen compound (XY), a nitrogen compound (Y), and hydroxide ions (OH⁻), by electrochemically reacting halogen and a nitrogen compound, in a reactor having cathode and anode electrodes, and including a solvent; and
a second operation of obtaining alkylene carbonate by mixing the mixture with alkylene (CₓH₂ₓ, where x is an integer of 2 to 10) and carbon dioxide.

2. The method for electrochemically synthesizing alkylene carbonate of claim 1, wherein, in the first operation, the mixture of a halogen-substituted nitrogen compound (XY), a nitrogen compound (Y), and hydroxyl ions (OH⁻) are obtained according to the following Scheme 1-1,
[Scheme 1-1] 4X- + 4HY = 2XY + 2H₂ + 2X- + 2Y-
in Scheme 1-1, X is halogen selected from the group consisting of fluorine, chlorine, bromine, and iodine,
Y is a nitrogen compound, and the nitrogen compound is a neutral nitrogen compound or an anionic nitrogen compound.

3. The method for electrochemically synthesizing alkylene carbonate of claim 1, wherein, in the second operation, alkylene carbonate is obtained according to the following Scheme 4, from halogenated alcohol and hydrogen carbonate ions (HCO₃⁻), respectively obtained according to the following Schemes 2, 3-1 and 3-2 by mixing the mixture of a halogen-substituted nitrogen compound, a nitrogen compound, and hydroxyl ions with alkylene and carbon dioxide,
[Scheme 2] 2alkene + 2XY + 2H₂O = 2halogenated alcohol + 2HY
[Scheme 3-1] 2Y- + 2CO₂ + 2H₂O = 2HY + 2HCO₃⁻
[Scheme 3-2] 2OH- + 2CO₂ = 2HCO₃⁻
[Scheme 4] 2HCO₃⁻ + 2halogenated alcohol = 2alkylene carbonate + 2X- + 2H₂O
in Schemes 2 to 4, X and Y are independently as defined in Scheme 1-1, where alkylene is selected from the group consisting of ethylene and propylene, halogenated alcohol is selected from the group consisting of halogenated ethanol and halogenated propanol, and alkylene carbonate is selected from the group consisting of ethylene carbonate and propylene carbonate.

4. The method for electrochemically synthesizing alkylene carbonate of claim 1, wherein the first operation and the second operation are performed simultaneously or performed sequentially.

5. The method for electrochemically synthesizing alkylene carbonate of claim 1, wherein an electrochemical reaction in the first operation is performed under conditions of 1 mA/cm² to 100 A/cm².

6. The method for electrochemically synthesizing alkylene carbonate of claim 1, wherein the nitrogen compound is a nitrogen compound of which conjugate acid (HY) of the nitrogen compound has a pKa of 7 to 14.

7. The method for electrochemically synthesizing alkylene carbonate of claim 6, wherein the nitrogen compound is selected from the group consisting of a compound having an imide functional group and an ammonium compound.

8. The method for electrochemically synthesizing alkylene carbonate of claim 6, wherein the nitrogen compound is at least one selected from the group consisting of succinimide, hydantoin, and cyanurate, and the ammonium compound is at least one selected from the group consisting of ammonium, monoethanolammonium, and diethanolammonium.

9. The method for electrochemically synthesizing alkylene carbonate of claim 1, wherein the solvent in the first operation is water.

10. The method for electrochemically synthesizing alkylene carbonate of claim 1, wherein the second operation is performed in at least one solvent selected from the group consisting of a polar organic solvent including alcohol, dimethylformamide, acetone, ether, and ester; and water.

11. The method for electrochemically synthesizing alkylene carbonate of claim 1, wherein ethylene and carbon dioxide in the second operation are mixed in a volume ratio of 1:0.5 to 2.

12. The method for electrochemically synthesizing alkylene carbonate of claim 1, wherein the first operation and the second operation are performed at a temperature within a range of higher than 0°C and 150°C or lower.
